# EUROPÄISCHE PATENTSCHRIFT

(11) **EP 0 567 857 B1**
(45) Veröffentlichungstag und Bekanntmachung des Hinweises auf die Patenterteilung: **22.11.1995**
(21) Anmeldenummer: 93106134.5
(22) Anmeldetag: 15.04.1993
(51) Int. Cl.: C07C 37/20, C07C 39/16

(54) **Bisphenol-Synthese an modifizierten Ionenaustauscherharzen unter Verwendung speziell gereinigter Carbonylverbindungen**
Bisphenol synthesis on modified ion-exchanger resins using especially purified carbonyl compounds
Synthèse de bisphénols sur des résines échangeurs d'ions modifiées en utilisant des composés carbonylés spécialement purifiés

(30) Priorität: 28.04.1992 DE 4213870
(43) Veröffentlichungstag der Anmeldung: 03.11.1993
(73) Patentinhaber: BAYER AG, 51368 Leverkusen (DE)
(72) Erfinder: Berg, Klaus, Dr., W-4150 Krefeld (DE); Fennhoff, Gerhard, Dr., W-4156 Willich 2 (DE); Pakull, Ralf, Dr., W-5000 Köln 71 (DE); Buysch, Hans-Josef, Dr., W-4150 Krefeld (DE); Wehrle, Bernhard, Dr., W-4018 Langenfeld (DE); Eitel, Alfred, Dr., W-4047 Dormagen (DE); Wulff, Klaus, Dr., W-4150 Krefeld (DE); Kirsch, Jürgen, Dr., W-5090 Leverkusen 1 (DE)

(56) Entgegenhaltungen:
- EP-A- 0 249 102
- US-A- 5 124 490

## Beschreibung

Die Erfindung betrifft die Synthese von Bisphenolen aus Monophenolen und Carbonylverbindungen wie Aldehyden und Ketonen an mit Alkyl-SH-Gruppen modifizierten sulfonsauren Ionenaustauschersystemen, wobei die verwendeten Monophenole und Carbonylverbindungen zuvor von direkt oder über Zwischenverbindungen alkylierend wirkenden Begleitsubstanzen gereinigt werden.

Die Kondensation von Phenolen und Carbonylverbindungen zur Bildung von Bisphenolen unter Verwendung verschiedener Katalysatoren wie beispielsweise Chlorwasserstoffsäure (US-A 21 82 308 und 21 91 831), Bortrifluorid (Chemical Abstracts 58, 3338c), Perchlorsäure (Chemical Abstracts 60, 1626h), Benzolsulfonsäure (Chemical Abstracts 59, 511h), verschiedenen Kationenaustauschharzen (z.B. GB-PC 842 209, 849 565 und 883 391) und auch schwefelhaltiger Verbindungen als Cokatalysatoren ist bekannt (z.B. US-A 24 68 982, 26 23 908, die Verwendung von Thioglykolsäure und 3-Mercaptopropionsäure, aus der US-A 27 75 620, der Zusatz von Alkylmercaptanen, aus Chemical Abstracts 58, 1403e, der Zusatz von Schwefelwasserstoff). Die bekannten schwefelhaltigen Cokatalysatoren können in der betrieblichen Praxis zu erheblichen Korrosionsschäden führen. Die mit diesen Cokatalysatoren hergestellten Bisphenole enthalten neben den nicht umgesetzten Edukten wie Monophenolen und Carbonylverbindungen, Reaktionswasser und zum gewünschten Bisphenol isomere Kondensationsprodukten bes. störende, korrosive und verfärbende Schwefelverbindungen, die nur schwer entfernt werden können.

Demgegenüber gestattet die Synthese von Bisphenolen mit phenolfeuchten, sulfonsauren Ionenaustauschern, bei denen cokatalytisch aktive Alkyl-SH-Gruppen an der Matrix fixiert sind, z.B. durch Aminoalkylmercaptan-Einheiten gemäß DE-A 3 619 450 oder durch Thiazolin-Einheiten gemäß DE.A 3 727 641, die Herstellung und Isolierung hochreiner Bisphenole.

Die Alkyl-SH-Gruppen (z.B. C₂-C₆-Alkyl, bevorzugt C₄-Alkyl) derartiger Harzsysteme können auch kovalent entweder an der Polystyrolmatrix oder partiell an den -SO₃H-Gruppen des Ionenaustauschers gebunden sein. Des weiteren kann es sich auch um eine Mischung beliebiger Zusammensetzung von reinem sulfonsauren Ionenaustauscherharz und einem Harz mit ausschließlich kovalent oder ionisch gebundenen Alkyl-SH-Gruppen handeln.

Bei der Herstellung von Bisphenolen aus Monophenolen und Carbonylverbindungen wie Aldehyden und Ketonen gemäß der DE-A 36 19 450 und der DE-A 37 27 641 ist jedoch ein rasches Abnehmen der Katalysatorreaktivität und -selektivität bereits nach relativ kurzen Produktionsphasen zu verzeichnen, so daß das eingesetzte Katalysatorsystem entweder regeneriert oder sogar vollständig erneuert werden muß. Dies führt zu Produktionsstillständen und zusätzlichem Mehraufwand in der Wartung der Produktionsanlagen.

Es wurde nun gefunden, daß die Katalysatorwirkung erheblich länger erhalten bleibt, die Raum/Zeitausbeute und Reinheit des Bisphenols und die Selektivität der Reaktion verbessert werden, wenn die als Edukte eingesetzten Monophenole und Carbonylverbindungen von alkylierend wirkenden Verbindungen gereinigt sind.

Gegenstand der Erfindung ist ein Verfahren zur Herstellung von Bis-(4-hydroxyphenyl)-alkanen aus Monophenolen und Carbonylverbindungen mittels Ionenaustauscher oder Mischungen von Ionenaustauschern, die vollständig oder partiell mit Alkyl-SH-Gruppen modifiziert worden sind, dadurch gekennzeichnet, daß die eingesetzten Monophenole und Carbonylverbindungen alkylierend wirkende Substanzen der Formel

R - X

worin
- X: für OH, Halogen, Carboxylat, Sulfat oder Sulfonat steht und
- R: ein offenkettiger, verzweigter oder unverzweigter, gesättigter oder ungesättigter C₁-C₆-Alkylrest oder ein C₅-C₇-cyclischer substituierter oder unsubstituierter, gesättigter oder ungesättigter Alkylrest sein kann,
in Mengen kleiner als 0,1 %, bevorzugt 0,05, besonders bevorzugt 0,01 % enthalten.

Alkylierend wirkende Substanzen im Sinne der Erfindung sind z.B. Alkohole, Alkylhalogenide, Carbonsäurealkylester, Alkylsulfate und Sulfonsäurealkylester.

Alkohole im Sinne der Erfindung sind beispielsweise Methanol, Ethanol, die Isomere des Propanols, die Isomere des Butanols, längerkettige, verzweigte oder unverzweigte Homologe, ungesättigte Alkohole wie beispielsweise Allylalkohol, cyclische gesättigte oder ungesättigte Alkohole wie beispielsweise Cyclopentanol, Cyclohexanol oder Cyclohexenol, mehrwertige Alkohole wie beispielsweise Ethylenglykol, 1,2-Propylglykol, 1,3-Propylglykol oder Glycerin.

Solche Verbindungen sind in der Regel in den Edukten von der Synthese her in wechselnden Mengen vorhanden.

Die Reinigung der Edukte vor ihrer Umsetzung kann mit den üblichen Methoden wie beispielsweise Destillieren, Umkristallisieren, Extrahieren oder mittels Molekularsieben durchgeführt werden, wobei die verbleibenden Mengen dieser Verbindungen <0,1 bzw. <0,05, besonders bevorzugt <0,01 % sein sollen.

### Beispiel 1

125 g phenolfeuchter Ionenaustauscher (Lewatit SC 102, Bayer AG), dessen Sulfonsäuregruppen zu 11,5 Mol-% mit 2-Aminoethylmercaptan belegt worden sind, werden in 499 g Phenol suspendiert. Danach wird in den auf 65°C temperierten Ansatz 29 g Aceton (mit 0,2 % H₂O, 0,01 % MeOH) hinzugegeben und 4 h lang gerührt. Anschließend wird der Acetonumsatz durch Messung des Restacetongehaltes im Ansatz nach Ketoximierungsmethode ermittelt (siehe Tabelle 1). Die Prozedur wird 20 mal wiederholt. Vor jedem neuen Ansatz wurde der Ionenaustauscher abfiltriert und mit geringfügigen Mengen Phenol produktfrei gespült. Danach wird wiederum die Umsatzaktivität des Ionenaustauschers gemessen (siehe Tabelle 1).

### Beispiel 2 (komparativ)

Wie Beispiel 1, nur daß, bezogen auf das eingesetzte Phenol, 5.000 ppm Methanol pro Ansatz zugesetzt wurden.

### Beispiel 3 (komparativ)

Wie Beispiel 2, nur daß, bezogen auf das eingesetzte Phenol, 5.000 ppm 2-Propanol zugesetzt wurden.

Die Umsatzaktivität (Acetonumsatz) des eingesetzten Ionenaustauschers nach 20 Ansätzen ist für drei Beispiele in Tabelle 1 aufgeführt.

**Tabelle 1**

| Ionenaustauscheraktivität der Beispiele 1 bis 3 (umgesetztes Aceton) | | |
|---|---|---|
| | nach 1 Ansatz | nach 20 Ansätzen |
| Beispiel 1 | 99,5 % | 98,7 % |
| Beispiel 2 | 98,5 % | 43,5 % |
| Beispiel 3 | 98,9 % | 75,2 % |

Bei allen Beispielen sind die %-Angaben Gewichts-% (Gew.-%), soweit nicht anders vermerkt.

## Patentansprüche

1. Verfahren zur Synthese von Bis-(4-hydroxyphenyl)-alkanen aus Monophenolen und Carbonylverbindungen mittels Ionenaustauscher oder Mischungen von Ionenaustauschern, die vollständig oder partiell mit Alkyl-SH-Gruppen modifiziert worden sind, dadurch gekennzeichnet, daß die eingesetzten Monophenole und Carbonylverbindungen alkylierend wirkende Substanzen der Formel
R - X,
worin
X für OH, Halogen, Carboxylat, Sulfat oder Sulfonat steht und
R ein offenkettiger, verzweigter oder unverzweigter, gesättigter oder ungesättigter C₁-C₆-Alkylrest oder ein C₅-C₇-cyclischer substituierter oder unsubstituierter, gesättigter oder ungesättigter Alkylrest sein kann,
in Mengen kleiner als 0,1 Gew.-%, enthalten.

2. Ein Verfahren gemäß Anspruch 1, dadurch gekennzeichnet, daß die Edukte Methanol und/oder Ethanol und/oder 1,2-Propanol und/oder 1,3-Propanol und/oder Isomere des Butanols und/oder Cyclohexanol in Mengen <0,01 Gew.-% enthalten.

3. Ein Verfahren gemäß Anspruch 1, dadurch gekennzeichnet, daß das eingesetzte Monophenol Phenol ist und die eingesetzte Carbonylverbindung Aceton ist.

## Claims

1. A process for the synthesis of bis-(4-hydroxyphenyl)-alkanes from monophenols and carbonyl compounds using ion-exchange materials or mixtures of ion-exchange materials, which have been completely or partly modified with alkyl-SH groups, characterised in that the monophenols and carbonyl compounds used contain alkylating substances of the formula
R - X
wherein
X represents OH, halogen, carboxylate, sulphate or sulphonate and
R may be an open-chain, branched or unbranched, saturated or unsaturated C₁-C₆ alkyl radical or a C₅-C₇ cyclic substituted or unsubstituted, saturated or unsaturated alkyl radical,
in amounts of less than 0.1 wt.%.

2. A process according to Claim 1, characterised in that the educts contain methanol and/or ethanol and/or 1,2-propanol and/or 1,3-propanol and/or isomers of butanol and/or cyclohexanol in amounts of < 0.01 wt.%.

3. A process according to Claim 1, characterised in that the monophenol used is phenol and the carbonyl compound used is acetone.

## Revendications

1. Procédé pour la synthèse de bis-(4-hydroxyphényl)-alcanes à partir de monophénols et de dérivés carbonylés à l'aide d'échangeurs d'ions ou de mélanges d'échangeurs d'ions modifiés en totalité ou en partie par des groupes alkyl-SH, caractérisé en ce que les monophénols et les dérivés carbonylés mis en oeuvre contiennent des substances à effet alkylant de formule :
R-X
dans laquelle
X représente OH, un halogène, un groupe carboxylate, sulfate ou sulfonate et
R représente un radical alkyle en C₁ à C₆ acyclique, ramifié ou droit, saturé ou insaturé, ou un radical alkyle cyclique en C₅-C₇, substitué ou non, saturé ou insaturé,
en quantités inférieures à 0,1 % en poids.

2. Procédé selon la revendication 1, caractérisé en ce que les produits de départ contiennent du méthanol et/ou de l'éthanol et/ou du 1,2-propanol et/ou du 1,3-propanol et/ou des isomères du butanol et/ou du cyclohexanol en quantités inférieures à 0,01 % en poids.

3. Procédé selon la revendication 1, caractérisé en ce que le monophénol mis en oeuvre est le phénol et le dérivé carbonylé mis en oeuvre est l'acétone.
